# DEMANDE DE BREVET EUROPEEN

(11) **EP 1 424 065 A1**
(43) Date de publication de la demande: **02.06.2004**
(21) Numéro de dépôt: 03292765.9
(22) Date de dépôt: 05.11.2003
(51) Int. Cl.: A61K 7/48, A61K 7/06, A61K 7/02

(54) **Composition biphase transparente pour application topique**

(30) Priorité: 26.11.2002 FR 0214827
(71) Demandeur: L'OREAL, 75008 Paris (FR)
(72) Inventeur: Cordier, Laurent, 06190 Roquebrune Cap Matin (FR)
(74) Mandataire: Rasson, Catherine

(57) **Abrégé**

L'invention concerne une composition pour application topique, constituée d'une phase aqueuse et d'une phase huileuse distinctes, la phase aqueuse comprenant au moins 40 % en poids d'un ou plusieurs polyols par rapport au poids total de la phase aqueuse et la phase huileuse comprenant au moins 5 % en poids d'une ou plusieurs huiles de silicone par rapport au poids total de la phase huileuse, la quantité de polyols et la quantité d'huiles de silicone étant telles que les indices de réfraction des deux phases sont substantiellement identiques.

Le polyol est choisi de préférence parmi la glycérine ou le dipropylène glycol ou un mélange de glycérine ou de dipropylène glycol et d'un autre polyol.

Les deux phases distinctes sont transparentes, et lors de l'agitation, ces deux phases transparentes forment une seule phase, transparente elle aussi.

L'invention concerne aussi l'utilisation de cette composition, dans le domaine cosmétique notamment pour le démaquillage, le nettoyage et/ou le soin de la peau, des lèvres et/ou des yeux ou pour le soin des cheveux.

## Description

La présente invention a pour objet une composition pour application topique, constituée de deux phases distinctes, une phase aqueuse et une phase huileuse, s'émulsionnant facilement par agitation en donnant une composition claire, c'est-à-dire transparente, et l'utilisation de la dite composition pour le démaquillage, le nettoyage et/ou le soin de la peau, des lèvres et/ou des yeux, et/ou pour le soin des cheveux.

Les compositions de ce type constituées de deux phases distinctes notamment d'une phase aqueuse et d'une phase huileuse sont généralement désignées sous le terme de "composition biphase". Elles se distinguent des émulsions par le fait qu'au repos, les deux phases sont distinctes au lieu d'être émulsionnées l'une dans l'autre. Ainsi, les deux phases sont séparées au repos par une seule interface, alors que, dans les émulsions, une des phases est dispersée dans l'autre sous forme d'une multitude de gouttelettes, et les interfaces sont donc multiples, ces interfaces étant généralement stabilisées par des tensioactifs émulsionnants et/ou des polymères émulsionnants. L'utilisation des compositions biphases nécessite une agitation préalable afin de former une émulsion extemporanée. Celle-ci doit être de qualité et de stabilité suffisantes pour permettre une application homogène des deux phases, mais telle qu'au repos, les deux phases se séparent rapidement et retrouvent leur état initial, ce phénomène étant plus connu sous le terme de "déphasage".

Des compositions biphases ont déjà été décrites par exemple dans les documents EP-A-370856 et EP-A-603080, notamment pour le démaquillage des yeux.

Les compositions biphases décrites jusqu'à présent forment après agitation, une émulsion opaque, mélange de deux phases non miscibles l'une dans l'autre. Or, ces compositions sont généralement présentées dans des récipients transparents, et l'opacité des deux phases émulsionnées est esthétiquement dommageable. En outre, on cherche de plus en plus à utiliser des compositions transparentes, car, tout comme l'eau, la transparence est le symbole de pureté et donc de propreté, et les compositions transparentes sont ainsi particulièrement appréciées des utilisateurs.

Toutefois, quand les deux phases ne sont pas miscibles, il est difficile d'obtenir un mélange de ces phases, qui soit transparent.

Il subsiste donc le besoin d'une composition biphase constituée de deux phases distinctes non miscibles, qui après agitation donne une émulsion transparente, tout en gardant les propriétés recherchées pour les compositions biphases, c'est-à-dire un déphasage rapide en deux phases transparentes.

De manière surprenante, la demanderesse a trouvé qu'il était possible d'obtenir une composition biphase transparente, qui, après agitation, donne une émulsion transparente et qui se déphase de nouveau rapidement en deux phases transparentes, en utilisant une phase aqueuse comprenant un ou plusieurs polyols en quantité suffisante et une phase huileuse comprenant une ou plusieurs huiles de silicone en quantité suffisante.

Plus particulièrement, l'invention a pour objet une composition pour application topique, constituée d'une phase aqueuse et d'une phase huileuse distinctes transparentes, la phase aqueuse comprenant au moins 40 % en poids d'un ou plusieurs polyols par rapport au poids total de la phase aqueuse, et la phase huileuse comprenant au moins 5 % en poids d'une ou plusieurs huiles de silicone par rapport au poids total de la phase huileuse, les quantités de polyols et d'huiles de silicone étant telles que les indices de réfraction des phases huileuse et aqueuse sont substantiellement égaux.

Il est connu d'introduire des polyols en une quantité assez importante dans des émulsions. Toutefois, il est surprenant que l'on puisse introduire une grande quantité de polyols dans des compositions biphases et que ces compositions après agitation restent stables pendant le temps d'utilisation, même en absence de tensioactifs. Par ailleurs, il est surprenant que l'interface reste nette et stable après retour au repos.

La composition selon l'invention étant destinée à une application topique, elle contient un milieu physiologiquement acceptable, c'est-à-dire compatible avec la peau, les muqueuses, les cheveux et le cuir chevelu.

La composition selon l'invention comprend au moins une phase aqueuse et une phase huileuse distinctes. Ces deux phases sont distinctes, c'est-à-dire qu'elles sont visibles l'une au-dessus de l'autre au repos. Elles sont transparentes au repos, et lorsque l'on agite la composition avant son utilisation, le mélange obtenu constitué de l'émulsion d'une phase dans l'autre est transparent. Les deux phases peuvent être ou non colorées.

Les indices de réfraction de la phase aqueuse et de la phase huileuse sont substantiellement égaux, le mot "substantiellement" signifiant que la différence entre les indices de réfraction des deux phases ne dépasse pas 0,005 et donc que les indices de réfraction des deux phases sont égaux à 0,005 près. Les indices de réfraction de ces phases sont généralement de l'ordre de 1,39 à1,47, ces indices étant mesurés à température ambiante (20 à 25°C).

Le mot « transparent » signifie que la composition a une turbidité inférieure ou égale à 300 NTU. La transparence d'une composition peut se mesurer par sa turbidité, et les NTU (Nephelometric Turbidity Units) sont les unités de mesure de la turbidité d'une composition. La mesure de turbidité peut être faite par exemple avec un turbidimètre model 2100P de la société HACH Compagny, les tubes utilisés pour la mesure étant référencés AR397A cat 24347-06. Les mesures sont effectuées à température ambiante (20°C à 25°C). La composition de l'invention a une turbidité allant généralement de 2 à 300 NTU, et de préférence de 5 à 200 NTU.

### Phase aqueuse

La phase aqueuse comprend au moins 40 % de polyols par rapport au poids total de la phase aqueuse. Ainsi, la quantité de polyols est de préférence d'au moins 10 % en poids par rapport au poids total de la composition, et elle peut aller par exemple de 10 à 90 % en poids et de préférence de 15 à 50% en poids par rapport au poids total de la composition.

Par "polyol", il faut comprendre toute molécule organique comportant au moins deux groupements hydroxyle libres. Comme polyols, on peut citer par exemple la glycérine, les glycols comme le butylène glycol, le propylène glycol, l'isoprène glycol, le dipropylène glycol, l'hexylène glycol et les polyéthylène glycols, le sorbitol, les sucres comme le glucose, et leurs mélanges. Selon un mode préféré de réalisation de l'invention, le polyol choisi est la glycérine, le dipropylène glycol ou leurs mélanges, ou un mélange de glycérine et/ou de dipropylène glycol et d'un ou plusieurs autres polyols notamment choisis parmi ceux indiqués ci-dessus : butylène glycol, propylène glycol, isoprène glycol, hexylène glycol, polyéthylène glycols, sorbitol, sucres, et leurs mélanges.

Outre les polyols, la phase aqueuse de la composition selon l'invention comprend de l'eau et tout additif hydrosoluble ou hydrodispersible. L'eau utilisée peut être de l'eau déminéralisée stérile et/ou une eau florale telle que de l'eau de rose, de l'eau de bleuet, de l'eau de camomille ou de l'eau de tilleul, et/ou une eau thermale ou minérale naturelle, comme par exemple : l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene. La phase aqueuse peut comprendre aussi de l'eau thermale reconstituée, c'est-à-dire une eau contenant des oligoéléments tels que zinc, cuivre, magnésium, etc.., reconstituant les caractéristiques d'une eau thermale.

La phase aqueuse peut comprendre aussi un alcool primaire, c'est-à-dire un alcool comportant de 1 à 6 atomes de carbone, tel que l'éthanol et l'isopropanol. Il s'agit de préférence de l'éthanol. Cet alcool peut être présent en une quantité allant par exemple de 0,01 à 30 % en poids et de préférence de 0,1 à 25 % en poids par rapport au poids total de la composition. L'ajout d'un tel alcool peut être notamment approprié lorsque la composition selon l'invention est utilisée comme produit pour le corps.

De préférence, le rapport pondéral entre la phase aqueuse et la phase huileuse va de 25/75 à 90/10, de préférence 30/70 à 70/30, mieux de 40/60 à 60/40, et encore mieux 45/55 à 55/45. La phase aqueuse représente donc généralement de 25 à 90 % en poids, de préférence de 30 à 70 % en poids, mieux de 40 à 60 % en poids et encore mieux de 45 à 55 % en poids par rapport au poids total de la composition.

### Phase huileuse

La phase huileuse représente généralement de 10 à 75 % et de préférence de 30 à 70 % en poids, mieux de 40 à 60 % en poids et encore mieux de 45 à 55 % en poids par rapport au poids total de la composition.

La phase huileuse de la composition selon l'invention comprend au moins 5 % d'une ou plusieurs huiles de silicone par rapport au poids total de la phase huileuse. Par rapport au poids total de la composition, la quantité d'huile(s) de silicone est de préférence d'au moins 0,5 % en poids, et cette quantité peut aller par exemple de 0,5 à 70 % en poids, de préférence de 5 à 65 % en poids, mieux de 10 à 60 % en poids et encore mieux de 20 à 60 % en poids par rapport au poids total de la composition.

Ces huiles de silicone peuvent être volatiles ou non volatiles. En outre, la phase huileuse peut comprendre une ou plusieurs autres huiles, volatiles ou non volatiles, comme indiqué ci-après, ainsi que des additifs liposolubles ou lipodispersibles.

Par huile de silicone, on entend une huile contenant au moins un atome de silicium, et notamment contenant des groupes Si-O. L'huile de silicone peut être choisie parmi les huiles de silicone non volatiles, les huiles de silicone volatiles et leurs mélanges.

L'huile de silicone volatile utilisable dans l'invention peut être choisie parmi les huiles de silicone ayant un point éclair allant de 40°C à 102°C, de préférence ayant un point éclair supérieur à 55°C et inférieur ou égal à 95°C, et préférentiellement allant de 65°C à 95°C. Comme huiles de silicone volatiles, on peut citer les huiles de silicones linéaires ou cycliques ayant de 2 à 7 atomes de silicium, ces silicones comportant éventuellement des groupes alkyle ou alkoxy ayant de 1 à 10 atomes de carbone. Comme exemples d'huiles de silicone volatiles, on peut citer notamment les cyclopolydiméthylsiloxanes (nom INCl : cyclomethicone), telles que le cyclopentasiloxane, le cyclohexasiloxane, l'octaméthylcyclotétrasiloxane, le décaméthylcyclopentasiloxane, le dodécaméthylcyclohexasiloxane ; les silicones linéaires telles que l'heptaméthylhexyltrisiloxane, l'heptaméthyloctyl-trisiloxane, l'hexaméthyl-disiloxane, l'octaméthyltrisiloxane, le décaméthyltétrasiloxane, le dodécaméthyl pentasiloxane ; et leurs mélanges.

L'huile de silicone non volatile utilisable dans l'invention peut être choisie parmi les polyméthylsiloxanes (PDMS), et les polyméthylsiloxanes phénylés tels que les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ; les polysiloxanes modifiés par des acides gras, des alcools gras ou des polyoxyalkylènes, et leurs mélanges.

Par ailleurs, la phase huileuse peut contenir un ou plusieurs autres huiles volatiles ou non choisies parmi les huiles hydrocarbonées, les huiles fluorées et leurs mélanges.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor; elle peut contenir des groupes ester, éther, amine, amide.

Par huile fluorée, on entend une huile contenant au moins un atome de fluor.

On peut citer par exemple comme huiles utilisables dans la composition de l'invention :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'amande d'abricot, de macadamia, d'arara, de coriandre, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle ;
- les hydrocarbures linéaires ou ramifiés, d'origine minérale ou synthétique, volatils ou non volatils, et leurs dérivés, comme l'huile de vaseline et le polyisobutène hydrogéné tel que l'huile de Parléam® ; les iso-alcanes (appelées aussi isoparaffines) en C₈-C₁₆, l'isododécane, l'isodécane, l'isohexadécane, comme par exemple les isoparaffines vendues sous les noms commerciaux Isopar par la société Exxon Chemical ou les huiles vendues sous les noms commerciaux Permethyl par la société Presperse ; et leurs mélanges ;
- des alcools gras ayant de 8 à 26 atomes de carbone, comme l'alcool cétylique, l'alcool stéarylique et leur mélange (alcool cétéarylique), l'octyldodécanol, le 2-butyloctanol, le 2-hexyldécanol, le 2-undécylpentadécanol ou l'alcool oléique ;

Selon un mode préféré de réalisation de l'invention, la composition contient, outre la ou les huiles de silicone, au moins une ou plusieurs huiles non volatiles choisies par exemple parmi les esters d'acides gras comportant de 8 à 29 atomes de carbone, tels que l'isononanoate d'isononyle, le myristate d'isopropyle, le palmitate d'isopropyle, le palmitate d'éthyl-2-hexyle, le stéarate d'octyl-2-dodécyle ; les hydrocarbures linéaires tels que l'huile de Parléam® et l'huile de vaseline ; les huiles hydrocarbonées d'origine végétale tells que l'huile d'amande d'abricot ; et leurs mélanges.

Dans la composition de l'invention, toutes huiles confondues, la quantité totale d'huile(s) non volatile(s) peut aller par exemple de 10 à 70 % en poids, et de préférence de 10 à 40 % en poids par rapport au poids total de la composition, et la quantité totale d'huiles volatiles peut aller par exemple de 5 à 50 % en poids, de préférence de 5 à 40 % en poids et mieux de 5 à 30 % en poids par rapport au poids total de la composition.

### Tensioactif

La composition biphase peut comprendre éventuellement au moins un tensioactif dans l'une ou l'autre des phases. Toutefois elle peut aussi être exempte de tensioactif. Quand elle contient un tensioactif, celui-ci peut être du type anionique, non ionique ou amphotère, mais il est de préférence du type non ionique et/ou anionique. Il est de préférence présent dans la phase aqueuse.

La quantité de tensioactif(s) en matière active doit être en une quantité telle que les deux phases restent distinctes au repos et ne se mélangent pas pour former une émulsion. Cette quantité doit généralement être inférieure ou égale à 1,5 % en poids par rapport au poids total de la composition. Elle peut aller par exemple de 0,01 à 1,5 % en poids, de préférence de 0,025 à 1 % en poids et mieux de 0,05 à 0,5 % en poids par rapport au poids total de la composition.

Parmi les agents tensioactifs non ioniques, ceux particulièrement préférés sont :
- les esters gras de sorbitol polyoxyéthylénés tels que le produit vendu sous la dénomination TWEEN 20 par la Société ICI.
- les alcools gras polyoxyéthylénés tels que le produit vendu sous la dénomination REMCOPAL 21912 AL par la Société GERLAND.
- les alkylphénols polyoxyéthylénés tels que le produit vendu sous la dénomination TRITON X 100 par la Société RÖHM-HAAS, et
- les condensats d'oxyde d'éthylène et d'oxyde de propylène tels que ceux vendus sous les dénominations SYNPERONIC PE par la Société ICI et en particulier ceux référencés L 31, L 64, F 38, F 88, L 92, P 103, F 108 et F 127.
- les diméthicone copolyols ou les mélanges les contenant tels que le produit vendu sous la dénomination DC 5225C par la société Dow Corning.

Parmi les agents tensioactifs anioniques, on peut notamment citer :
- les alkylsulfates, les alkyl éther sulfates et leurs sels, notamment leurs sels de sodium, comme le mélange de Sodium Laureth Sulfate / Magnesium Laureth Sulfate / Sodium Laureth-8 Sulfate / Magnesium Laureth-8 Sulfate, vendu sous le nom de Texapon ASV par la société Henkel ; le lauryl éther sulfate de sodium (C12-14 70/30) (2,2 OE) commercialisé sous les dénominations SIPON AOS 225 ou TEXAPON N702 PATE par la société Henkel, le lauryl éther sulfate d'ammonium (C12-14 70/30) (3 OE) commercialisé sous la dénomination SIPON LEA 370 par la société Henkel ; l'alkyl (C12-C14) éther (9 OE) sulfate d'ammonium commercialisé sous la dénomination RHODAPEX AB/20 par la société Rhodia Chimie ;
- les alkylsulfoacétates tels que celui vendu sous la dénomination LATHANOL LAL par la Société STEPAN ;
- les sulfosuccinates d'alkyle, par exemple le mono-sulfosuccinate d'alcool laurylique (C12/C14 70/30) oxyéthyléné (3 OE) commercialisé sous les dénominations SETACIN 103 SPECIAL, REWOPOL SB-FA 30 K 4 par la société Witco, le sel di-sodique d'un hemi-sulfosuccinate des alcools C12-C14, commercialisé sous la dénomination SETACIN F SPECIAL PASTE par la société Zschimmer Schwarz, l'oléamidosulfosuccinate di-sodique oxyéthyléné (2 OE) commercialisé sous la dénomination STANDAPOL SH 135 par la société Henkel, le mono-sulfosuccinate d'amide laurique oxyéthyléné (5 OE) commercialisé sous la dénomination LEBON A-5000 par la société Sanyo, le sel di-sodique de mono-sulfosuccinate de lauryl citrate oxyéthyléné (10 OE) commercialisé sous la dénomination REWOPOL SB CS 50 par la société Witco, le sel disodique de mono-sulfosuccinate de mono-éthanolamide ricinoléique commercialisé sous la dénomination REWODERM S 1333 par la société Witco ;
- les polypeptides qui sont obtenus par exemple par condensation d'une chaîne grasse sur les aminoacides de céréale et notamment du blé et de l'avoine, comme par exemple le sel de potassium de la lauroyl protéine de blé hydrolysée, commercialisé sous la dénomination AMINOFOAM W OR par la société Croda, le sel de triéthanolamine de cocoyl protéine de soja hydrolysée, commercialisé sous la dénomination MAY-TEIN SY par la société Maybrook, le sel de sodium des lauroyl amino-acides d'avoine, commercialisé sous la dénomination PROTEOL OAT par la société Seppic, l'hydrolysat de collagène greffé sur l'acide gras de coprah, commercialisé sous la dénomination GELIDERM 3000 par la société Deutsche Gelatine, les protéines de soja acylées par des acides de coprah hydrogénés, commercialisées sous la dénomination PROTEOL VS 22 par la société Seppic ;
- Les dérivés des aminoacides, par exemple parmi les sarcosinates et notamment les acylsarcosinates comme le lauroylsarcosinate de sodium commercialisé sous la dénomination SARKOSYL NL 97 par la société Ciba ou commercialisé sous la dénomination ORAMIX L 30 par la société Seppic, le myristoyl sarcosinate de sodium, commercialisé sous la dénomination NIKKOL SARCOSINATE MN par la société Nikkol, le palmitoyl sarcosinate de sodium, commercialisé sous la dénomination NIKKOL SARCOSINATE PN par la société Nikkol ; les alaninates comme le N-lauroyl-N-methylamidopropionate de sodium commercialisé sous la dénomination SODIUM NIKKOL ALANINATE LN 30 par la société Nikkol ou commercialisé sous la dénomination ALANONE ALE par la société Kawaken, et le N-lauroyl N-methylalanine triéthanolamine, commercialisé sous la dénomination ALANONE ALTA par la société Kawaken ; les N-acylglutamates comme le mono-cocoylglutamate de triéthanolamine commercialisé sous la dénomination ACYLGLUTAMATE CT-12 par la société Ajinomoto, et le lauroyl-glutamate de triéthanolamine commercialisé sous la dénomination ACYLGLUTAMATE LT-12 par la société Ajinomoto ; les aspartates comme le mélange de N-lauroylaspartate de triéthanolamine et de N-myristoylaspartate de triéthanolamine, commercialisé sous la dénomination ASPARACK LM-TS2 par la société Mitsubishi ; les dérivés de la glycine, comme le N-cocoylglycinate de sodium et le N-cocoylglycinate de potassium tels que les produits commercialisés sous les dénominations AMILITE GCS-12 et AMILITE GCK-12 par la société Ajinomoto ;
- les sulfonates, par exemple les alpha-oléfines sulfonates comme l'alpha-oléfine sulfonate de sodium (C14-16) commercialisé sous la dénomination BIO-TERGE AS-40 par la société Stepan, commercialisé sous les dénominations WITCONATE AOS PROTEGE et SULFRAMINE AOS PH 12 par la société Witco
   ou commercialisé sous la dénomination B10-TERGE AS-40 CG par la société Stepan, l'oléfine sulfonate de sodium secondaire commercialisé sous la dénomination HOSTAPUR SAS 30 par la société Clariant ; les alkyl aryl sulfonates linéaires comme le xylène sulfonate de sodium commercialisé sous les dénominations MANROSOL SXS30, MANROSOL SXS40, MANROSOL SXS93 par la société Manro ;
- les iséthionates, notamment les acyliséthionates comme le cocoyliséthionate de sodium, tel que le produit commercialisé sous la dénomination JORDAPON Cl P par la société Jordan.

Parmi les tensioactifs amphotères ou zwitterioniques, on peut notamment citer :
- les dérivés alkylamido alkylamines tels que le N-cocoyl-N-carboxyméthoxyéthyl-N-carboxyméthyl-éthylènediamine N-di-sodique (nom CTFA : Disodium cocoampho-diacetate) commercialisé en solution aqueuse saline sous la dénomination MIRANOL C2M CONC NP par la société Rhodia Chimie ; le N-cocoyl-N-hydroxyéthyl-N-carboxyméthyl-éthylènediamine N-sodique (nom CTFA : sodium cocampho-acetate) et le mélange d'éthanolamides d'acide de coco (nom CTFA : Cocamide DEA) ;
- les bétaïnes, comme par exemple la cocobétaïne comme le produit commercialisé sous la dénomination DEHYTON AB-30 par la société Henkel, la laurylbétaïne comme le produit commercialisé sous la dénomination GENAGEN KB par la société Clariant, la laurylbétaïne oxyethylénée (10 OE), comme le produit commercialisé sous la dénomination LAURYLETHER(10 OE)BETAINE par la société Shin Nihon Rica, la stéarylbétaïne oxyéthylénée (10 OE) comme le produit commercialisé sous la dénomination STEARYLETHER(10 OE)BETAINE par la société Shin Nihon Rica ;
- les alkylamidopropylbétaïnes et leurs dérivés comme par exemple la cocamidopropyl bétaine commercialisée sous la dénomination LEBON 2000 HG par la société Sanyo, ou commercialisée sous la dénomination EMPIGEN BB par la société Albright & Wilson, la lauramidopropyl bétaïne commercialisée sous la dénomination REWOTERIC AMB12P par la société Witcotels que la cocamidopropylbétaïne comme les produits vendus sous les dénominations TEGO BETAINE par la Société GOLDSCHMIDT ;
- les dérivés d'imidazoline tels que le produit vendu sous la dénomination CHIMEXANE HD par la Société CHIMEX, et
- leurs mélanges.

### Adjuvants

La composition selon l'invention peut également contenir des adjuvants ou additifs cosmétiques conventionnels qui se trouveront dans l'une ou l'autre phase selon leur nature hydrophile ou lipophile, tels que par exemple des parfums, des agents conservateurs et bactéricides, des colorants, des agents adoucissants, des tampons, des humectants, des filtres U.V. (ou filtres solaires), des électrolytes tel que le chlorure de sodium ou un ajusteur de pH par exemple acide citrique ou hydroxyde de sodium), et leurs mélanges.

Comme conservateurs, on peut utiliser tout conservateur habituellement utilisé dans les domaines considérés, tels que par exemple les parabens et le gluconate de chlorhexidine.

Comme bactéricide, on peut par exemple utiliser un mono(C₃-C₉)alkyl-ou (C₃-Cg)alcényléther de glycérol dont la fabrication est décrite dans la littérature, en particulier dans E. Baer, H.O.L. Fischer - J. Biol. Chem. 140-397-1941. Parmi ces mono(C₃-C₉)alkyl-ou (C₃-C₉)alcényléthers de glycérol, on utilise de préférence le 3-[(2-ethylhexyl)oxy]-1,2-propanediol, le 3-[(heptyl)oxy]-1,2-propanediol, le 3-[(octyl)oxy]-1,2-propanediol et le 3-[(allyl)oxy]-1,2-propanediol. Un mono(C₃-Cg)alkyléther de glycérol plus particulièrement préféré selon la présente invention est le 3-[(2-ethylhexyl)oxy]-1,2-propanediol, vendu par la société SCHULKE & MAYR G.m.b.H. sous la dénomination commerciale SENSIVA SC 50 (nom INCl : Ethylhexylglycerin).

Parmi les agents adoucissants, on peut en particulier citer l'allantoïne et le bisabollol, les planctons, et certains extraits de plantes comme les extraits de rose et les extraits de mélilot.

Selon l'invention, la composition peut comprendre en outre de préférence dans la phase aqueuse, un agent de déphasage en une proportion allant par exemple de 0,025 à 5 % en poids par rapport au poids total de la composition.

Comme agent de déphasage, on peut citer par exemple les chlorures d'alkyldiméthylbenzylammonium comme décrit dans le document EP-A-603080, et notamment le chlorure de benzalkonium, et les mélanges le contenant ; les alkyl glucosides alcoxylés comportant un groupe d'ammonium quaternaire et notamment le chlorure de lauryl methyl gluceth-10 hydroxypropyldimonium, comme décrit dans le document EP-A-847746 ; les polymères et copolymères de vinylpyrrolidone et notamment le copolymère de polyvinylpyrrolidone / hexadecene comme décrit dans le document WO-A-99/56704 ; et leurs mélanges.

Quand un tel agent est présent, le rapport entre l'agent tensioactif et l'agent de déphasage va de préférence de 0,005/1 à 200/1 et mieux de 0,01/1 à 120/1.

Comme actifs utilisables dans la composition de l'invention, on peut citer par exemple, les enzymes (par exemple lactoperoxydase, lipase, protéase, phospholipase, cellulases) ; les flavonoïdes ; les agents hydratants tels que les hydrolysats de protéines ; le hyaluronate de sodium ; les anti-inflammatoires ; les oligomères procyannidoliques ; les vitamines comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; l'urée ; la caféine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; l'hydrocortisone ; la mélatonine ; les extraits d'algues, de champignons, de végétaux, de levures, de bactéries ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents tenseurs ; les céramides ; les huiles essentielles ; et leurs mélanges; et tout actif approprié pour le but final de la composition.

Les filtres U.V. utilisables dans la composition de l'invention sont organiques. Ils peuvent être présents en une quantité en matière active allant de 0,01 à 20 % en poids de matière active, de préférence de 0,1 à 15 % en poids, et mieux 0,2 à 10 % en poids par rapport au poids total de la composition.

Comme exemples de filtres organiques, actifs dans l'UV-A et/ou l'UV-B, pouvant être ajoutés dans la composition de l'invention, on peut citer par exemple, les dérivés à fonction sulfonique tels que les dérivés sulfonés ou sulfonatés du benzylidène camphre, de la benzophénone ou du phénylbenzimidazole, plus particulièrement les dérivés du benzylidène camphre, comme l'acide benzène 1,4 [di(3-méthylidène-campho 10-sulfonique)], (nom INCl : Terephthalylidene Dicamphor Sulfonic Acid) fabriqué sous le nom « MEXORYL SX » par la société CHIMEX. l'acide 4'-sulfo 3-benzylidènecamphre (nom INCl : Benzylidene Camphor Sulfonic Acid), fabriqué sous le nom « MEXORYL SL» par la société CHIMEX, l'acide 2-[4-(camphométhylidène) phényl] benzimidazole-5-sulfonique, l'acide phénylbenzimidazole sulfonique (nom INCl : Phenylbenzimidazole Sulfonic Acid), commercialisé sous le nom EUSOLEX 232 par la société MERCK ; les dérivés de l'acide para-aminobenzoique ; les dérivés salicyliques tels que le salicylate d'éthyl hexyle vendu sous le nom commercial NEO HELIOPAN OS par Haarmann et Reimer ; les dérivés du dibenzoylméthane tels que le Butyl Methoxydibenzoylmethane vendu notamment sous le nom commercial PARSOL 1789 par Hoffmann La Roche ; les dérivés cinnamiques tels que l'éthylhexyl Methoxycinnamate vendu notamment sous le nom commercial PARSOL MCX par Hoffmann La Roche ; les dérivés de β,β'-diphénylacrylate tels que l'octocrylene (α-cyano-β,β-diphénylacrylate de 2-éthylhexyle) vendu sous le nom commercial UVINUL N539 par la société BASF ; les dérivés de la benzophénone tels que la Benzophenone-1 vendu sous le nom commercial UVINUL 400 par BASF, la Benzophenone-2 vendu sous le nom commercial UVINUL D50 par BASF, la Benzophenone-3 ou Oxybenzone, vendu sous le nom commercial UVINUL M40 par BASF, la Benzophenone-4 vendu sous le nom commercial UVINUL MS40 par BASF ; les Dérivés du benzylidène camphre tels que le 4-Methylbenzylidene camphre vendu sous le nom commercial EUSOLEX 6300 par MERCK ; les dérivés du phenyl benzimidazole tels que le Benzimidazilate vendu sous le nom commercial NEO HELIOPAN AP par Haarmann et Reimer ; les dérivés de la triazine tels que l'Anisotriazine vendu sous le nom commercial TINOSORB S par CIBA GEIGY et l'éthylhexyl triazone vendu notamment sous le nom commercial UVINUL T150 par BASF ; les dérivés du phenyl benzotriazole tels que Drometrizole Trisiloxane vendu sous le nom commercial SILATRIZOLE par Rhodia Chimie ; les dérivés anthraniliques tels que le Menthyl anthranilate vendu sous le nom commercial NEO HELIOPAN MA par Haarmann et Reimer ; les dérivés d'imidazolines ; les dérivés du benzalmalonate ; et leurs mélanges.

Les compositions décrites ci-dessus peuvent être conditionnées, de façon connue, dans un flacon à un seul compartiment. L'utilisateur doit alors agiter le flacon avant d'en verser le contenu sur un coton. On peut également prévoir que les deux phases de la composition soient introduites dans deux compartiments indépendants d'un même flacon, un système étant prévu pour leur mélange au moment de la distribution. De tels dispositifs sont décrits par exemple dans les documents EP-A-497256 et FR-A-2697233.

La composition selon l'invention peut être utilisée pour toute application topique, notamment elle peut constituer une composition cosmétique ou dermatologique. Elle peut en particulier être utilisée pour le soin, le nettoyage et/ou le démaquillage de la peau, les lèvres et /ou des yeux, et également comme composition pour le soin des cheveux.

L'invention a aussi pour objet l'utilisation cosmétique d'une composition cosmétique telle que définie ci-dessus pour le soin, le démaquillage et/ou le nettoyage de la peau, des lèvres et/ou des yeux, et/ou pour le soin des cheveux.

La présente invention a encore pour objet un procédé cosmétique de démaquillage, de nettoyage et/ou de soin de la peau, des lèvres et/ou des yeux, caractérisé par le fait que l'on applique sur la peau, les lèvres et/ou les yeux, une composition cosmétique telle que définie ci-dessus.

La présente invention a aussi pour objet un procédé cosmétique de soin des cheveux, caractérisé par le fait que l'on applique sur les cheveux, une composition cosmétique telle que définie ci-dessus.

L'exemple ci-après de compositions selon l'invention est donné à titre d'illustration et sans caractère limitatif. Les quantités y sont données en % en poids, sauf mention contraire.

### Exemple 1 : composition démaquillante

### Phase huileuse

- Cyclopentasiloxane 14 %
- Isododécane 15 %
- Palmitate d'isopropyle 19,5994 %
- parfum 0,2 %
- Colorant 0,0006 %
- Ethylhexyloxyglycerin (Sensiva SC 50) 1,2 %

### Phase aqueuse

- Glycérine 19,93 %
- Dipropylène glycol 12,92 %
- Triéthanolamine (neutralisant) 0,07 %
- Methylparaben 0,2 %
- Colorant 0,00015 %
- Chlorure de sodium 0,5 %
- Disodium EDTA (chélatant) 0,08 %
- Eau déminéralisée qsp 100 %

Mode opératoire : On mélange les constituants de la phase huileuse d'une part et ceux de la phase aqueuse d'autre part. Puis on mélange les deux phases.

On obtient une composition qui, au repos, comporte une phase aqueuse et une phase huileuse distinctes colorées et transparentes. Par agitation, les deux phases donnent une composition transparente, permettant un bon démaquillage de la peau, des lèvres et des yeux.

### Exemple 2 : composition de soin de la peau

### Phase huileuse

- Cyclopentasiloxane 23 %
- Polyisobutène hydrogéné 14,5 %
- Huile d'amande d'abricot (Prunus Armeniaca kernel Oil) 12,5 %

### Phase aqueuse

- Dipropylène glycol 45,5 %
- Chlorhexidine digluconate 0,1 %
- Colorant (Blue 1) 0,00015 %
- Eau déminéralisée 4,39985 %

Mode opératoire : On mélange les constituants de la phase huileuse d'une part et ceux de la phase aqueuse d'autre part. Puis on mélange les deux phases.

On obtient une composition comportant une phase aqueuse et une phase huileuse distinctes transparentes. Par agitation, les deux phases donnent une composition transparente. Cette composition peut être utilisée notamment pour le soin de la peau.

### Exemple 3 : composition pour le corps

### Phase huileuse

- Cyclopentasiloxane 44,1 %
- Isononanoate d'isononyle 3,5 %
- Huile végétale 1 %
- parfum 0,4 %
- Acétate de tocophérol 0,5 %
- Ethylhexyloxyglycerine (Sensiva SC 50) 0,5 %

### Phase aqueuse

- Glycérine 20 %
- Propylène glycol 1 %
- Panthénol 0,3 %
- Colorant 0,0001 %
- Ethanol 18 %
- Parfum 0,15 %
- Eau déminéralisée qsp 100 %

### Mode opératoire :

On mélange les constituants de la phase huileuse d'une part et ceux de la phase aqueuse d'autre part. Puis on mélange les deux phases.

On obtient une composition comportant une phase aqueuse et une phase huileuse distinctes transparentes. Par agitation, les deux phases donnent une composition transparente. Cette composition peut être utilisée notamment pour le soin de la peau.

## Revendications

1. Composition pour application topique, constituée d'une phase aqueuse et d'une phase huileuse distinctes transparentes, la phase aqueuse comprenant au moins 40 % en poids d'un ou plusieurs polyols par rapport au poids de phase aqueuse et la phase huileuse comprenant au moins 5 % en poids d'une ou plusieurs huiles de silicone par rapport au poids de phase huileuse, les quantités de polyols et d'huiles de silicone étant telles que les indices de réfraction des phases huileuse et aqueuse sont substantiellement égaux.

2. Composition selon la revendication précédente, **caractérisée en ce que** la quantité de polyol(s) va de 10 à 90 % en poids et de préférence de 15 à 50 % en poids par rapport au poids total de la composition.

3. Composition selon la revendication 1 ou 2, **caractérisée en ce que** le polyol est choisi parmi la glycérine, les glycols, le sorbitol, les sucres, et leurs mélanges.

4. Composition selon la revendication précédente, **caractérisée en ce que** les glycols sont choisis parmi le butylène glycol, le propylène glycol, l'isoprène glycol, le dipropylène glycol, l'hexylène glycol et les polyéthylène glycols.

5. Composition selon l'une quelconque des revendications précédentes, **caractérisée en ce que** le rapport pondéral entre la phase aqueuse et la phase huileuse va de 40/60 à 60/40, et de préférence 45/55 à 55/45.

6. Composition selon l'une quelconque des revendications précédentes, **caractérisée en ce que** la quantité d'huile(s) de silicone va de 0,5 à 70 % en poids et de préférence de 5 à 65 % en poids par rapport au poids total de la composition.

7. Composition selon l'une quelconque des revendications précédentes, **caractérisée en ce que** l'huile de silicone est choisie parmi les huiles de silicone non volatiles, les huiles de silicone volatiles, et leurs mélanges.

8. Composition selon l'une quelconque des revendications précédentes, **caractérisée en ce que** la phase huileuse comprend en outre au moins une huile non volatile choisie parmi les esters d'acide gras comportant de 8 à 29 atomes de carbone, les huiles hydrocarbonées d'origine végétale, les hydrocarbures linéaires, et leurs mélanges.

9. Composition selon l'une quelconque des revendications précédentes, **caractérisée en ce qu'**elle comprend au moins un tensioactif choisi parmi les tensioactifs anioniques, non ioniques et amphotères.

10. Composition selon l'une quelconque des revendications précédentes, **caractérisée en ce que** le tensioactif est présent en une quantité allant de 0,01 à 1,5% en poids par rapport au poids total de la composition.

11. Composition selon l'une quelconque des revendications précédentes, **caractérisée en ce qu'**elle comprend en outre au moins un agent de déphasage en une proportion allant de 0,025 à 5 % en poids par rapport au poids total de la composition.

12. Composition selon l'une quelconque des revendications précédentes, **caractérisée en ce qu'**elle constitue une composition cosmétique ou dermatologique.

13. Utilisation cosmétique d'une composition cosmétique selon l'une quelconque des revendications 1 à 11, pour le soin, le démaquillage et/ou le nettoyage de la peau, des lèvres et/ou des yeux et/ou pour le soin des cheveux.

14. Procédé cosmétique de démaquillage, de nettoyage et/ou de soin de la peau, des lèvres et/ou des yeux, **caractérisé par le fait que** l'on applique sur la peau, les lèvres et/ou les yeux, une composition cosmétique selon l'une quelconque des revendications 1 à 11.

15. Procédé cosmétique de soin des cheveux, **caractérisé par le fait que** l'on applique sur les cheveux, une composition cosmétique selon l'une quelconque des revendications 1 à 11.
